# EUROPEAN PATENT SPECIFICATION

(11) **EP 2 076 860 B1**
(45) Date of publication and mention of the grant of the patent: **16.11.2016**
(21) Application number: 07818517.0
(22) Date of filing: 27.09.2007
(51) Int. Cl.: G06F 19/00, G06K 9/62

(54) **FEATURE SELECTION ON PROTEOMIC DATA FOR IDENTIFYING BIOMARKER CANDIDATES**
MERKMALAUSWAHL AUF PROTEOMISCHEN DATEN ZUM IDENTIFIZIEREN VON BIOMARKIERUNGSKANDIDATEN
SÉLECTION DE CARACTÉRISTIQUES SUR DES DONNÉES PROTÉOMIQUES POUR IDENTIFIER DES BIOMARQUEURS CANDIDATS

(30) Priority: 28.09.2006 EP 06020405
(43) Date of publication of application: 08.07.2009
(73) Proprietor: Private Universität für Gesundheitswissenschaften Medizinische Informatik und Technik - UMIT, 6060 Hall in Tirol (AT)
(72) Inventor: PLANT, Claudia, 80809 München (DE); BAUMGARTNER, Christian, 6065 Thaur (AT); TILG, Bernhard, 6094 Axams (AT)
(74) Representative: Vossius & Partner Patentanwälte Rechtsanwälte mbB
(86) International application number: PCT/EP2007/008434
(87) International publication number: WO 2008/037479

(56) References cited:
- WO-A-2005/011474
- US-A1- 2002 095 260
- VAN DIJCK G ET AL: "Hierarchical feature subset selection for features computed from the continuous wavelet transform" 2005 IEEE WORKSHOP ON MACHINE LEARNING FOR SIGNAL PROCESSING (IEEE CAT. NO.05TH8851C) IEEE PISCATAWAY, NJ, USA, 2005, pages 86-91, XP002464773 ISBN: 0-7803-9518-2
- GOH LIANG ET AL: "An integrated feature selection and classification method to select minimum number of variables on the case study of gene expression data." JOURNAL OF BIOINFORMATICS AND COMPUTATIONAL BIOLOGY OCT 2005, vol. 3, no. 5, October 2005 (2005-10), pages 1107-1136, XP008087338 ISSN: 0219-7200
- CLAUDIA PLANT ET AL: "Feature Selection on High Throughput SELDI-TOF Mass-Spectrometry Data for Identifying Biomarker Candidates in Ovarian and Prostate Cancer" DATA MINING WORKSHOPS, 2006. ICDM WORKSHOPS 2006. SIXTH IEEE INTERNATIONAL CONFERENCE ON, IEEE, PI, December 2006 (2006-12), pages 174-179, XP031010232 ISBN: 0-7695-2702-7
- WANG Y ET AL: "Gene selection from microarray data for cancer classification-a machine learning approach" COMPUTATIONAL BIOLOGY AND CHEMISTRY, ELSEVIER, vol. 29, no. 1, February 2005 (2005-02), pages 37-46, XP004744785 ISSN: 1476-9271
- FUKUNAGA K: "Introduction to Statistical Pattern Recognition, Second Edition" 1990, ACADEMIC PRESS , XP002464774 page 491, paragraph 4 - page 497, paragraph 1

## Description

The present invention generally relates to data mining for cancer identification and particularly to feature selection on proteomic data such as high-throughput mass spectrometry data for identifying biomarker candidates.

The identification of putative proteomic marker candidates is a big challenge for the biomarker discovery process.

Pathologic states within cancer tissue may be expressed by abnormal changes in the protein and peptide abundance. By the availability of modem high throughput techniques such as SELDI-TOF (surface-enhanced laser desorption and ionization time-of-flight) mass spectroscopy a large amount of high dimensional mass spectrometric data is produced from a single blood or urine sample. Each spectrum is composed of peak amplitude measurements at approximately 15,200 features represented by a corresponding mass-to-charge ratio (m/z value). For early stage ovarian cancer detection traditional single biomarkers, such as the widely used cancer antigen 125 (CA125) can only detect 50%-60% of patients with stage I ovarian cancer [10]. Analogously, the single use of the prostate specific antigen (PSA) value for early stage prostate cancer identification is not specific enough to reduce the number of false positives [16]. The analysis of high dimensional serum proteomic data gives a deeper insight into the abnormal protein signaling and networking which has a high potential to identify previously not discovered marker candidates.

More precisely, there are two aims for mining proteomic data: one subtask is to identify diseased subjects with the highest possible sensitivity and specificity. For this purpose, classification algorithms, e.g. support vector machines (SVM), neural networks and the K-nearest neighbor classifier (K-NN) can be applied. In the training phase, these algorithms are trained on a training data set which contains instances labeled according to classes, e.g. healthy and diseased, and then predict the class label of novel unlabeled instances [13].

The second subtask is the identification of unknown marker candidates for cancer. This subtask goes beyond classification towards to understanding the underlying bio-molecular mechanisms of the disease. Feature selection methods, which try to find the subset of features with the highest discriminatory power, can be applied for this purpose.

Numerous feature selection strategies for classification have been proposed, for a comprehensive survey see e.g. [7]. Following a common characterization, it is distinguished between filter and wrapper approaches.

### Filters

Filter approaches use an evaluation criterion to judge the discriminating power of the features. Among the filter approaches, it can further be distinguished between rankers and feature subset evaluation methods. Rankers evaluate each feature independently regarding its usefulness for classification. As a result, a ranked list is returned to the user. Rankers are very efficient, but interactions and correlations between the features are neglected. Feature subset evaluation methods judge the usefulness of subsets of the features. The information of interactions between the features is in principle preserved, but the search space expands to the size of O(2^{d}). For high-dimensional proteomic mass spectra, only very simple and efficient search strategies, e.g. forward selection algorithms, can be applied because of the performance limitations. More details on two rankers and two subset evaluation methods are given in the following.

**Rankers.** The information gain [17] of an attribute reflects how much information the attribute provides on the class label of the objects. For numerical attributes, the data set is first discretized using the method of Fayyad and Irani [5]. The information gain of a feature *f*ᵢ with respect to class *cⱼ* is defined as |G(*f*ᵢ) = H(*cⱼ*) - H(*cⱼ*|*f*ᵢ), whereas H(*cⱼ*) is the entropy of the class c*ⱼ*, H(*f*ᵢ) is the entropy of the attribute *f*ᵢ and H(c*ⱼ* | *f*ᵢ) the conditional entropy. Relief is an instance based attribute ranking method that has been introduced by Kira and Rendell [8] and has been extended to the method reliefF for multi-class and noisy data sets by Kononenko [9]. The main idea of relief is that useful attributes have significantly different values for instances of different classes and similar values for instances of the same class. To compute a relevance score for an attribute, for randomly sampled instances the K-nearest neighbors of the same class and from each of the other classes are taken into account.

**Feature subset evaluation.** Correlation-based feature selection (CFS) [6] is a feature subset evaluation heuristic which assigns high scores to feature subsets which show a high correlation with the class attribute and a low correlation among each other. Redundant features are rejected because they are highly correlated with other features, but only under the assumption that they get into a common subset during the search process. Consistency-based subset evaluation [11] uses an evaluation metric that favors attribute subsets which achieve a strong single class majority. This means, the attribute subsets are evaluated with respect to their discriminatory power regarding the majority class. The idea is to find different small attribute subsets to separate the different classes from each other.

### Wrappers

The wrapper attribute selection method uses a classifier to evaluate attribute subsets. Cross-validation is used to estimate the accuracy of the classifier on novel unclassified objects. For each examined attribute subset, the classification accuracy is determined. Adapted to the special characteristics of the classifier, in most cases wrapper approaches identify attribute subsets with a higher classification accuracy than filter approaches, cf. [7]. As the attribute subset evaluation methods, wrapper approaches can be used with an arbitrary search strategy. Among all feature selection methods, wrappers are the most computational expensive ones, due to the use of a learning algorithm for each examined feature subset.

Due to the high dimensionality of the data, most classification algorithms cannot be directly applied on proteomic mass spectra. One reason is the so-called curse of dimensionality: With increasing dimensionality the distances among the instances assimilate. Noisy and irrelevant features further contribute to this effect, making it difficult for the classification algorithm to establish decision boundaries. A further reason why classification algorithms are not applicable on the full dimensional space are performance limitations. Ultimately, feature transformation techniques are applied before classification, e.g. in [18].

Furthermore, also for the task of identifying unknown marker candidates, the use of traditional methods is limited due to the high dimensionality of the data. Moreover, feature transformation is not useful as well because the marker candidates can not be identified out of the transformed features.

There is therefore a need for an improved method for feature selection on proteomic data, in particular for a method which allows for efficient identification of feature subsets which allow for a high classification accuracy.

The present invention provides a method for feature selection on proteomic data, in particular, high-throughput mass spectroscopy data from, for example, SELDI-TOF mass spectroscopy for identifying biomarker candidates. The method can be used for cancer identification, in particular ovarian or prostate cancer. The method of the present invention is a three-step feature selection framework, which advantageously combines elements of existing feature selection methods, preferably combining the efficiency of the filter approaches with the effectiveness of wrapper approaches and exploiting special characteristics of the data.

The method according to the present invention specifically includes the steps of (a) removing irrelevant features, (b) selecting the best-ranked features, and (c) selecting the best region representatives. The method according to the invention is based on the use of a ranker, such as, for example, information gain or reliefF, a classifier, such as, for example, a support vector machine, a neural network or a K-nearest neighbor classifier, and an arbitrary search strategy. The search strategy may be a ranked search wherein, in each step, the feature with the lowest rank is removed and the classification accuracy is evaluated using the classifier. The search strategy may also be simulated annealing which, during the search, can leave a found local maximum and continue the search for a global maximum. The search strategy may preferably be a modified binary search, an efficient search strategy which is able to identify the smallest set of features that establishes a classification accuracy which is close to the optimum.

In the method of the present invention, the dimensionality, that is the number of features is reduced in each step while the classification accuracy is at least maintained.

The present invention thus provides a method which is a generic framework for feature selection using a classifier, a search strategy and a ranker. The method is efficient and applicable on very high dimensional proteomic data sets. The classification results on the selected features which will be presented in the following confirm and outperform the results reported in literature on the ovarian and the prostate data set.

The present invention will now be described in more detail with reference to the drawings, in which:
- Fig. 1: shows an overview of the method according to the present invention;
- Fig. 2: illustrates the distribution of the ranked features using the information gain or reliefF as a ranker;
- Fig. 3: shows the classification accuracy over the number of ranked features in the search space used in step (b),
- Fig. 4: shows an exemplary pseudocode for the algorithm which may be used for the modified binary search,
- Fig. 5: shows an exemplary pseudocode which may be used for the step selecting the best region representatives,
- Fig. 6: shows some selected regions selected in the step of selecting the best region representatives on the ovarian data set,
- Fig. 7: shows some selected regions selected in the step of selecting the best region representatives on the prostate data set, and
- Fig. 8: shows, in more detail, a selected region on the prostate data set.

In the following, the feature selection method according to the present invention is evaluated on two SELDI-TOF mass spectroscopy (MS) data sets which are available at the website of the US National Cancer Institute: http://home.ccr.cancer.gov/ncifdaproteomics/ppatterns.asp.

Both data sets derive from clinical studies with the objective to identify putative biomarker candidates by comparing well defined groups of cancer versus healthy controls. Each spectrum is composed of d = 15,154 features.

### Ovarian Data Set

The ovarian data set 8-7-02 contains 162 instances of ovarian cancer and 91 instances of a healthy control group. The data set is an improved version of the data set published in [15], using a WCX2 protein chip and robotic automation.

Trajanowski et al. [18] recently proposed an approach for ovarian cancer identification based on dimensionality reduction. They use a multi-step feature transformation technique based on wavelet transformation and present the results on this data set and on a high resolution data set on ovarian cancer. With 2-fold cross validation, an accuracy of 91.12% is reported for SVM on the wavelet reduced 8-7-02 data set. 100% accuracy can be achieved on 9 key features selected by the framework of the invention using a linear SVM and 10-fold cross validation. Even on the full dimensional space, the classification accuracy is with 99.60% much higher than on the wavelet reduced data set. This indicates that the data quality is very high. Alexe et al. [2] analyzed this data set using a combinatorics and optimization-based methodology. With a system of 41 rules they achieved a sensitivity and specificity of 100% on this data set.

### Prostate Data Set

This data set consists of four classes:
- c1: healthy, with a PSA value < 1 ng/mL (63 instances),
- c2: healthy, PSA value > 4 ng/mL (190 instances),
- c3: prostate cancer, PSA value 4-10 ng/mL (26 instances),
- c4: prostate cancer, PSA value > 10 ng/mL (43 instances).

In [16] this data set has been analyzed with ProteinQuest, a tool combining genetic algorithms and cluster analysis. The same algorithm has also been applied in [15] and [4] for feature selection for ovarian cancer data sets. Conrads et al. used in [4] a data set very similar to the 8-7-02 data set. However, the number of instances differ.

The genetic algorithm starts with randomly selected feature subsets. A fitness criterion rating the ability of the feature subsets to generate class-pure clusters is used. The best feature subsets are refined with the genetic algorithm. As optimal discriminating pattern the m/z values 2,092, 2,367, 2,582, 3,080, 4,819, 5,439 and 18,220 have been identified. This method achieves to identify prostate cancer with 94.74% accuracy (accuracy on the class corresponding to c3 u c4) and 77.73% percent of the instances were correctly identified to have benign conditions (accuracy on c1 ∪ c2). However, especially for class c2, i.e. healthy men with marginally elevated PSA levels, the reported specificity is with 71 % quite low. Due to different methodology and validation methods, the results of Protein-Quest are not directly comparable to our technique. Nevertheless, we obtain with 78.8 % a much higher specificity of class c2 and an overall accuracy of 97.83% on this data set. On the selected features by the method of the invention, linear SVM correctly identified instances with prostate cancer with 97.10% accuracy (accuracy on c3 ∪ c4) and even 99.60% of the instances were correctly identified to have benign conditions (accuracy on c1 ∪ c2).

Some notations which are frequently used in the following: Data sets DS with an initial dimensionality *d* are considered. Besides of *d* numerical features for each instance *I*, a categorical class label *class(I)* is given. Within the feature selection method of the present invention, the resulting data set of step i is denoted by *resᵢ* with the classification accuracy *accᵢ* and the dimensionality *dimᵢ.* A set of features is denoted by *F* = *{f1, ...fi}*. A classification algorithm is denoted by C, which can be an arbitrary classifier, e.g. a support vector machine. Additionally, a ranker R is used, which is a feature selection method generating a ranked list of the features and associating a quality value to each feature. The rank of fi is denoted by *rank(fi)* and the quality of *fi* by *quality(fi).* Furthermore, the index, i.e. the position of the m/z value of the feature *fi* in the original data set DS, is denoted by *index(fi).* The feature with the smallest m/z value has the index 1, the feature with the largest m/z value has the index *d*.

The three-step framework of the method according to the present invention is a hybrid method combining the good performance of a filter approach with the advantages of the wrapper approach: more accustomed to the learning algorithm the wrapper approach achieves better results in most cases. The framework uses a classifier C e.g. SVM and a ranker R, e.g. information gain and a search strategy S. An optimal feature subset for biomarker identification and diagnosis is a subset consisting of as few features as possible and achieving highest classification accuracy. C, R, S and special properties of proteomic data are used for an effective and efficient exploration of the huge search space of 2d feature subsets to find a close to optimal solution.

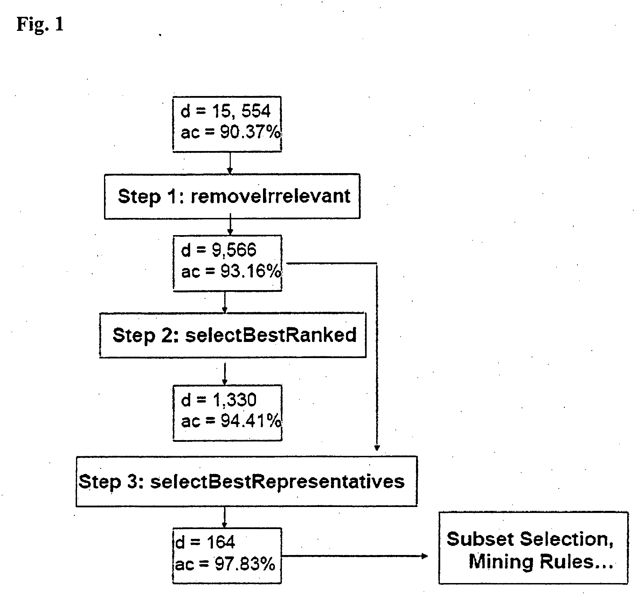

Figure 1 gives an overview of the steps in the feature selection framework according to the invention. The classifier, the evaluation criterion and the search strategy can be arbitrarily chosen, also depending on time and memory resources. Starting with the full data set as input, the feature selection process selects and removes in each step noisy, redundant or irrelevant features, while keeping the accuracy at least constant.

In the following, the single steps are discussed in detail. Results are given for SVM, K-NN as classifiers, information gain and reliefF as rankers, and ranked search, simulated annealing, and a novel heuristic called modified binary search as search strategies. For the classifiers the implementations of the WEKA machine learning package[1] are used. For the nearest neighbor classifier, K=5 nearest neighbors are used. A linear SVM with the standard parameterization in WEKA of c = 1.0 and γ = 0.01 is used. To estimate the classification accuracy, 10-fold cross validation is used.

### Step 1: Removing Irrelevant Features

High dimensional proteomic data contains noisy features and moreover a good portion of the features is irrelevant for diagnosis. In the first step, the irrelevant features are to be identified and discarded using the ranker R and the classifier C. To get a baseline for the classification accuracy, first the accuracy on the full feature space is determined using C. For ovarian data 99.60% is achieved with linear SVM, for prostate data 90.37%. Then, the evaluation criterion is used to remove all irrelevant features. For information gain this means all features with information gain 0 are removed and the accuracy is determined again. For the ovarian data set *dim1* = 6,238 attributes remain and the accuracy stays the same, i.e. *acc1* = 99.60. For prostate data, the reduction to *dim1* = 9,566 attributes improves the classification accuracy to *acc1* = 93.16%.

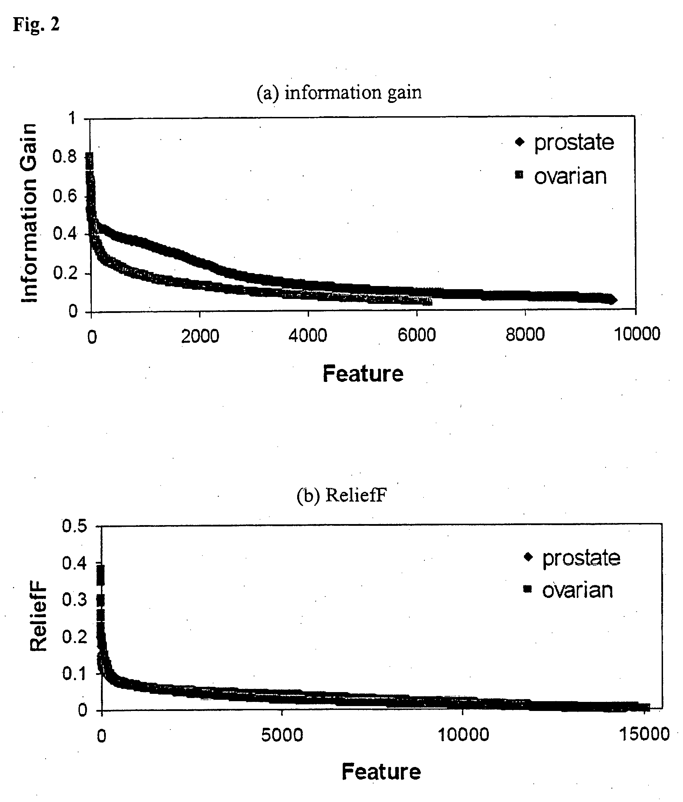

Figure 2(a) shows for each feature the quality according to information gain and Figure 2(b) according to relief for both data sets. Only features with information gain and reliefF > 0 are depicted and the features are displayed in the ranked order according their score with respect to R. For both data sets and both evaluation criteria the feature ranking shows a characteristic property: There are only very few highly ranked features and many features that are irrelevant or almost irrelevant. For the ovarian data set this aspect is even more evident and the feature ranking shows a perfect exponential distribution. This indicates that much more features can be removed without affecting the accuracy. In the following the discussion is focused on the use of information gain as ranker and SVM as classifier. More results for reliefF and 5-NN are given in the section Results below.

### Step 2: Selecting the Best Ranked Features

After having identified and discarded the irrelevant features using R in the last step, the dimensionality should be further reduced without affecting the accuracy, i. e. the aim is to identify a feature subset *res2* with *acc2* >= *acc1* and *dim2 <= dim1.* Since *dim1* is still in the order of magnitude of several thousands of features, the search space is restricted to the ranked list generated by R in this step. This means, the search space is reduced to the size of O(*d*). It should be noted that the features discarded now may be re-included in the following step. The only features which are definitely discarded are those features discarded in the previous step, i.e. the features which have been rated as completely irrelevant by R. An arbitrary search strategy S and the classifier C are used to find a smaller attribute subset while keeping the accuracy at least constant. During the search, all intermediate results of S are evaluated by C. In the following, three different search strategies will be discussed in combination with SVM after the information gain has been used as ranker R and to discard the irrelevant features.

### Ranked Search

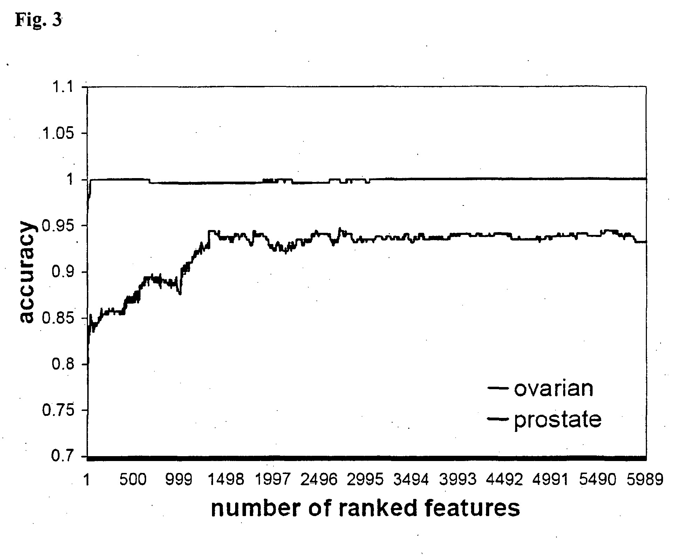

Starting with *res1*, in each step the ranked search removes the feature with the lowest rank and evaluates the classification accuracy using C. Figure 3 shows the classification accuracy for SVM on both data sets for the 6,000 top ranked features, whereas the ranking has been generated using information gain. For the ovarian data set, 100% accuracy is achieved using the 38 top ranked attributes. For the prostate data set there are many local maxima of the accuracy. The global maximum of 94.72% is reached using the 2,722 top ranked attributes. Although the search space is restricted to a size of 0(d), ranked search is very inefficient because only one feature is removed in each step and it is required to run the classifier *dim1-*times.

### Simulated Annealing

Simulated annealing has been successfully applied for solving complex global optimization problems in various application areas, such as shortest-path problems [12] or e.g. for object recognition in images [3], or scheduling of tasks in distributed systems [14]. The inspiration behind this method is derived from annealing in metallurgy, a technique involving heating and controlled cooling of a material to increase the size of its crystals and reduce their defects. Unlike hill-climbing methods, simulated annealing can leave a found local maximum and continue the search for a global maximum. The algorithm mainly uses 4 parameters: The temperature T, an interval δ, a cooling strategy for decreasing δ and T, i.e. δ ↓ and T↓ and starting point Ps. Starting with Ps, in each step the algorithm replaces the current solution with a random solution chosen out of the interval δ. In each step, the interval decreases by δ ↓ and the temperature by T↓. The algorithm iterates until the temperature has decreased to zero. During the run of the algorithm, the amount of randomness decreases and the result is refined.

Simulated annealing is applied as search strategy S with the parameters T = 40, δ = 3,000, δ ↓ = 75, T↓ = 1 and Sp = 2,080 on the ovarian data set. 40 features with an accuracy of 100% are selected. For the prostate data set, T = 40, δ = 3,200, δ ↓ = 80, T↓ = 1 and Sp = 3,198 is chosen, resulting in 2,800 features and an accuracy of 94.09 %. The starting point Sp has been chosen as 1/3 * dim1, with δ and δ ↓ correspondingly adjusted. The result and the runtime of simulated annealing strongly depends on suitable parameter settings.

### Modified Binary Search

Ranked search, and also simulated annealing are quite inefficient, because these methods produce many immediate results which have to be evaluated by the classifier C. In addition, suitable parameter settings are crucial for simulated annealing. Moreover, it is not required to find the global maximum accuracy in the search space of the ranked features (which can be only guaranteed by using ranked search). Restricting the search space to the ranked features means rating single features only and neglecting dependencies and interactions between the features. Therefore, for complex data sets satisfactory results in terms of accuracy can not be expected from the result of this step. As a input for the next step it is sufficient to identify the smallest set of features that establish a classification accuracy which is close to the optimum in the search space of this step.

For this purpose, binary search may be applied, which is very efficient (O(log(*d*)): Starting with *res1* the 1/2 * *dim1* lower ranked features and determine the accuracy *acchalf* are removed. If *acchalf* is higher than *acc1*, searching is continued in the left half, otherwise, it is moved to the right. For the ovarian data set, this strategy works well, since there are not many local maxima of the accuracy, cf. Figure 3. The binary search identifies the same 40 features as simulated annealing in 3 minutes. For the prostate data set the search takes 17 minutes due to many local maxima and 2,539 features are identified with an accuracy of 94.09%. This result is better than the one of simulated annealing, because it contains fewer features with the same accuracy. However, the binary search in principle can get stuck at every small local maximum in a region with many features. To avoid this, the search is guided towards feature sets which are as small as possible.

Starting with an empty attribute subset, the accuracy shows a steep rise while the very best ranked features are added, cf. Figure 3. The accuracy then reaches a level at which it keeps at most constant while adding more and more features according their ranked order. In the end, a slow decrease in accuracy can be observed. Figure 3 shows only the accuracy for the 6,000 best ranked features, so the decrease can not be observed for the ovarian data set. There are two reasons for this observation: The features added first are also the features providing the maximal amount of information on the class labels of the objects. Later added features are less likely to contain novel information, as they are worse ranked and the curse of dimensionality makes it more difficult for the classifier to establish decision boundaries. The goal of the algorithm is to find the point where the accuracy reaches the plateau which, of course, can contain many local maxima and minima.

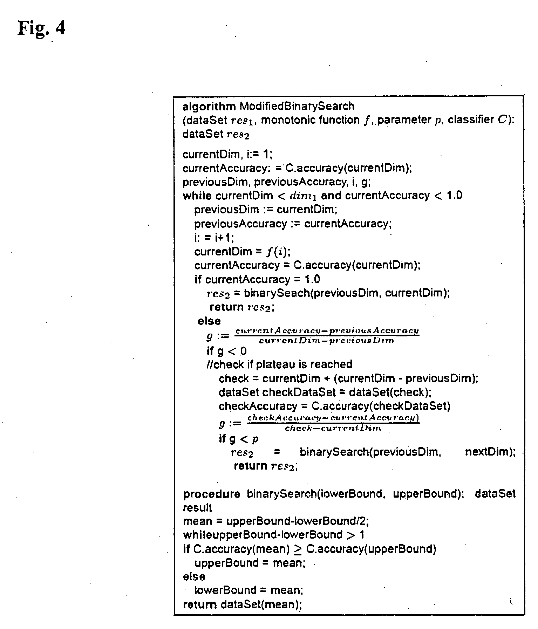

The algorithm preferably used in the method according to the present invention divides the search space into intervals of monotonically increasing size and determines points at which the accuracy is evaluated (if the algorithm does not stop the search earlier). Intervals of monotonically increasing size are advantageously used because of the steep increase of the accuracy at the beginning which is flattening later. Then, the gradient between the accuracy of adjacent points is determined. If the gradient is negative, the accuracy is still fast increasing. It is then checked if the plateau is reached soon by looking forward one step of the current interval size. If a flattening of the accuracy can be observed, the desired point in the interval between the current upper bound and the upper bound of the look-ahead-interval has been found. A binary search in this region is performed and the found feature subset is reported as result res2. The algorithm also terminates searching if the maximum accuracy of 100% is reached for the current point. In this case, the algorithm tries to reduce the dimensionality by performing binary search in the interval between the current and the last point. Pseudocode for the algorithm is given in Figure 4. The algorithm takes as input the data set from the previous step res1, a monotonically increasing function f to determine the size of the intervals, a parameter p that controls how much further increase in accuracy we allow after we reached the plateau and the classifier C. For f in principle all monotonically increasing functions can be used. In the experiments, good results were obtained using the cubic increasing function f(x) = x3. The parameter p avoids that the algorithm can not detect the plateau because of random fluctuations of the accuracy that occur in complex data sets, such as the prostate data set after the plateau has been reached. This parameter should be set as the maximal estimated contribution of the local maxima and minima to the overall accuracy. In all experiments, p is set to 0.1. Table 1 provides a comparison among simulated annealing (SA), binary search (BS) and modified binary search (MBS). The search strategies were implemented in Java.

Table 1 shows the number of selected attributes, the classification accuracy using linear SVM and the runtime in minutes on a PC with a 2.99 GHz CPU and 0.99 GB RAM. On both data sets modified binary search is the fastest method. Modified binary search is even faster than binary search because in MBS binary search is only performed within small interval of the feature list. Starting with an empty feature set, this interval can be efficiently determined. Moreover, MBS finds the smallest feature subsets providing the highest classification accuracy on both data sets.

**Table 1. Comparison of Search Strategies.**

| DS | SA | BS | MBS |
|---|---|---|---|
| ovarian | 40 | 39 | 39 |
| | 100.0% | 100.0% | 100.0% |
| | 6.6 min | 2.6 min | 0.4 min |
| prostate | 2,800 | 2,539 | 1,331 |
| | 94.09% | 94.09% | 94.41% |
| | 41.1 min | 17.5 min | 12.3 min |

### Step 3: Selecting the Best Region Representatives

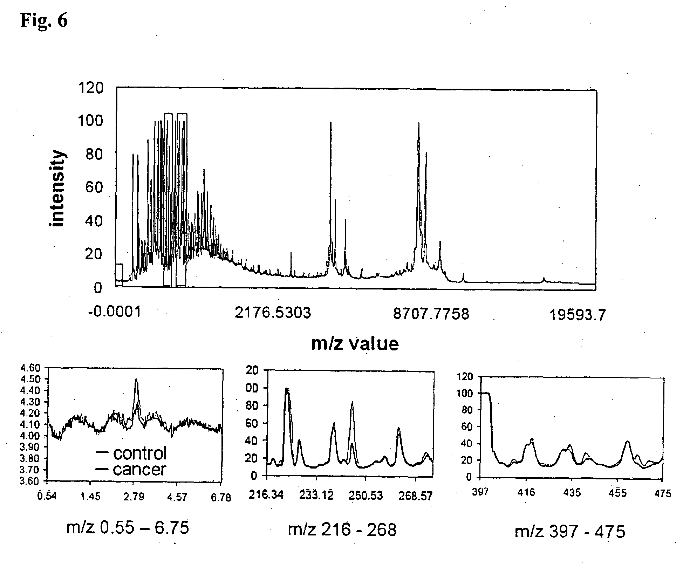

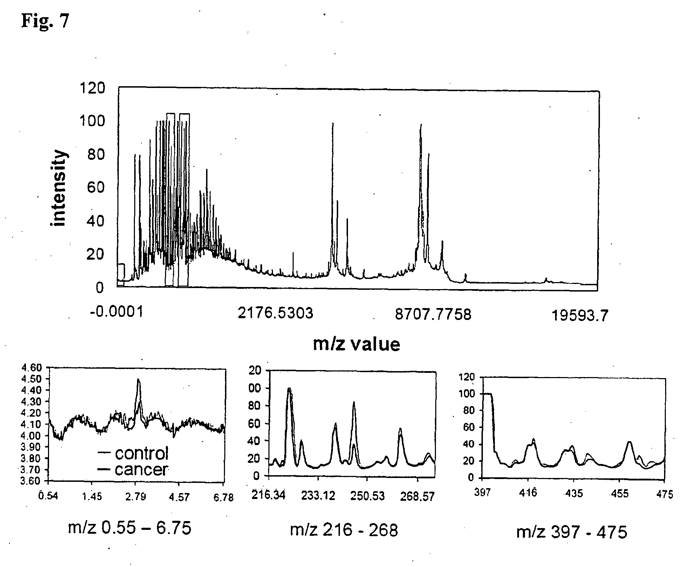

Typical peaks in SELDI-TOF-MS data sets consist of continuous ranges of features (cf. Figure 6, 7). The resulting set res2 contains features which have been highly ranked by R. If a region, lets say a peak, of the spectrum differentiates well among the classes, the features of this region are highly ranked and are all included in res2. However, as they are highly correlated, most of them are redundant because they represent the same information. On the other hand, there may be under-represented regions, which consist of not so highly ranked features which can contain valuable different information. As input, the two previous result sets *res2* and *res1* are used. In this step, first the redundant features are removed from *res2* and then, optionally features from *res1* are added for under-represented regions if this leads to a further improvement of the accuracy.

### Removing Redundant Features

Many of the features contained in res2 are redundant and can be removed. The idea is that every region of the spectrum has to be represented with as few as possible features having the highest discriminating power. In this step, a forward selection method is used, that exploits the consistency of proteomics spectra data which is also assumed in binning. Proteomic mass spectra are often binned using a function with a linear increasing bin width. This means, in the in the area of lower m/z values, fewer features are represented as one bin and in the area of high m/z values, many features can be merged into one bin [4]. This is due to the fact that in many different fragments of peptides with low molecular weight are causing many narrow peaks the region of low m/z values. In the region of higher m/z values, whole peptides leading to broader peaks can be identified. The following simple linear increasing function b may be used, which can be called *binning function,* to find a first approximation of a reasonable region size.

### Definition 1(Binning Function) Lets ε N. The binning function b is defined as b(fᵢ) = max(1, index(fᵢ)/100 * s)

For a feature *fi, b* determines a region size of s% of the index of *fi.* The minimum region size is set to 1. The following reports the results using res2 obtained with SVM and information gain and modified binary search as search strategy. For each region the best ranked feature is chosen as representative, and C is used to evaluate the accuracy. For the ovarian data set 9 features and an accuracy of 100% are obtained, that is, the maximum accuracy has been achieved. For the prostate data set, this results in 19 features and an accuracy of 93.48%. Since the accuracy declined from originally 94.41% on 1,331 attributes, the attribute which is best ranked among the remaining attributes is subsequently added in each step for each region, and the accuracy with C is evaluated again. For 187 attributes the accuracy of 94.41% is reached again. More precisely, the algorithm for adding the best representatives works as follows. It is started with an empty result data set *res3.* The list of ranked features of *res2* is first sorted by the index of the features. Then, the region size of each feature using b is determined, and it is determined if there are any better ranked features within the region of *fi* in *res2.* If not, *fi* is added to the result set. In each step, the algorithm adds for each region the best feature among the remaining features until no further improvement of accuracy can be achieved.

### Adding Missing Region Representatives

Different regions of the spectra tend to contain different information. Some of the regions in the intermediate result set may be under-represented or not represented at all, since *res2* has already been a drastically reduced attribute set containing only high ranked features. Therefore, also the list of ranked features of *res1* is used. As described above, for each not represented region the best representative is determined using the binning function. This is added as long as an improvement of the accuracy can be obtained.

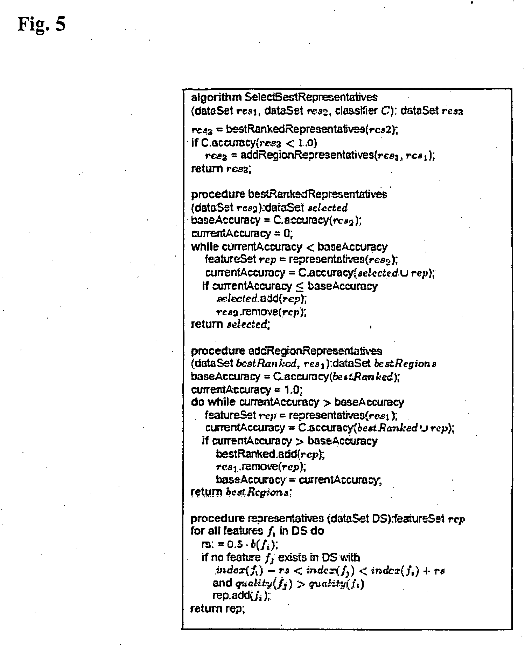

The pseudocode for the whole algorithm is depicted in Figure 5. The method *representatives()* selects for each region the best representative which has not been selected before. As a final step (which has been left out in the pseudocode for simplicity), it is tested if there are redundant features. More precisely, the list of features sorted with respect to their index is taken. Then it is tried to leave out for each pair of neighboring features the feature which has been lower ranked by R and evaluate the accuracy again. For the prostate data set, this results in 164 features and a final accuracy of 97.83%. The confusion matrix for linear SVM and 10-fold cross validation shows only seven classification errors, whereas four of them are due to confusing the two different stages of prostate cancer.

| | | | |
|---|---|---|---|
| 63 | 0 | 0 | 0 |
| 0 | 189 | 1 | 0 |
| 0 | 1 | 22 | 3 |
| 0 | 1 | 1 | 41 |

In contrast to correlation based feature selection (CFS), this algorithm does not only cover linear dependencies among the features. Similar as in the wrapper approach, the classification algorithm is used to evaluate the intermediate results. Only features that are useful for the special classifier are included in the result set. For linear SVM redundant features are almost synonymous with highly linearly correlated features, but this may be different for a neural network. The algorithm is efficient: For the prostate data set, step 3 takes 5.30 minutes, whereas for the ovarian data set we are done in 50 seconds.

### Results

Table 2 summarizes the results using linear SVM as classifier and the information gain as ranker. All three single steps of our method reduce the dimensionality, and at the same time improve the classification accuracy for this combination of C and R. For comparison, in Table 3 the results for 5-NN as C and reliefF as R are given. The classification accuracy using 5-NN on the full feature space is lower than the accuracy using SVM on both data sets. Also for reliefF and 5-NN our method achieves a sound reduction of features and improvement in classification accuracy. However, for 5-NN and reliefF on both data sets, the final set of features is larger and the classification accuracy is lower than with information gain and SVM. Also, not all the single steps lead to an improvement with respect to both aspects, the classification accuracy and the number of features. However always at least one of these two aspects is improved in each single step. In principle, every combination of R, C and S can be applied.

**Table 2. Linear SVM and Information Gain**

| DS | full space | step 1 | step 2 | step 3 |
|---|---|---|---|---|
| ovarian | 15,154 | 6,238 | 39 | 9 |
| | 99.60% | 99.60% | 100.0% | 100% |
| prostate | 15,154 | 9,566 | 1,331 | 164 |
| | 90.37% | 93.16% | 94.41% | 97.83% |

**Table 3. 5-NN and ReliefF**

| DS | full space | step 1 | step 2 | step 3 |
|---|---|---|---|---|
| ovarian | 15,154 | 15,037 | 66 | 90 |
| | 93.28% | 93.20% | 99.20% | 99.40% |
| prostate | 15,154 | 14,435 | 35 | 361 |
| | 87.27% | 87.27% | 85.09% | 92.50% |

Figure 6 depicts some selected regions that have been identified by the feature selection framework on the ovarian data set. A randomly selected spectrum of the control group with highlighted regions is depicted. Below the highlighted regions are shown in more detail comparing the healthy instance to a randomly selected instance with ovarian cancer. A majority of the relevant features can be found in the region of low m/z values, this has also been stated in [2]. The 9 features found using SVM and information gain are the m/z values 2.76, 25.49, 222.42, 244.66, 261.58, 417.73, 435.07, 464.36 and 4,002.46. Besides the m/z value 2.78 all these features have also been selected using reliefF and 5-NN. Among the 90 selected features with reliefF and 5 NN, 70% percent represent m/z values below 3,000.

For the prostate data set, also in the area of higher m/z values discriminating regions have been found. Figure 7 shows some selected regions. Out of the 164 selected features using SVM and information gain, the most evident changes between healthy and diseased instances can be observed in the regions representing the m/z values of approximately 500, 5,000 and 9,000. For clarity reasons, one randomly selected spectrum of class c1 (healthy, PSA < 1 ng/mL) is compared to one randomly selected spectrum of class c4 (prostate cancer, PSA > 10 ng/mL) with respect to the three highlighted regions in Figure 7. Most of the features selected by reliefF and 5-NN are also in these three areas. Besides this, more features in the region of very low m/z values have been selected using reliefF and 5-NN.

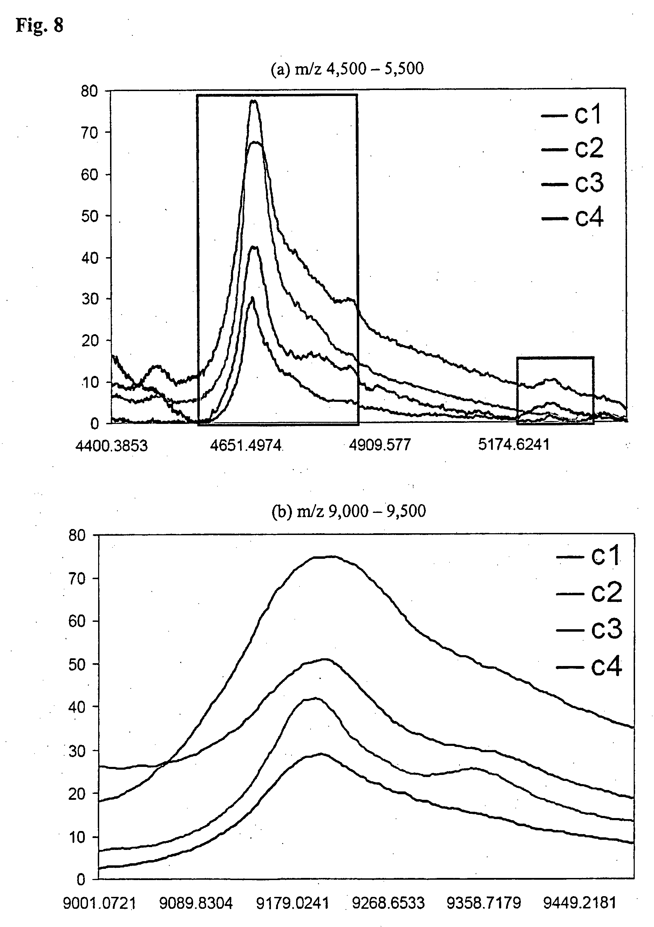

In Figure 8 the interesting regions from m/z 4,500 to 5,500 and m/z 9,000 to 9,500 are inspected in more detail with respect to all classes by depicting one randomly selected spectrum of each class. In Figure 8(a) there are two interesting regions which are highlighted: One is the peak between approximately m/z 4,550 and 4,850. The amount of the corresponding peptides is considerably lower for the instances with prostate cancer (c3 and c4) than for the instances with benign conditions (c1 and c2). The other interesting region is a peak of smaller intensity at approximately m/z of 5,250. Here the amount of the corresponding peptides is increased for the instances of the class c4 (prostate cancer, highly elevated PSA value) and c2 (healthy, elevated PSA value) with respect to class c1. The same region is also displayed in more detail in Figure 7 for an instance of class c1 compared to an instance of class c4. It is especially interesting, because in most of the discriminating regions, the abundance of peptides is reduced for the instances with cancer (also on the ovarian data set, cf. Figure 6).

In Figure 8(b) it can be seen that the abundance of the peptides corresponding to the m/z values around 9,200 is reduced for the instance of prostate cancer with a highly elevated PSA value (class c4) with respect to the class of the healthy control group without elevation of the PSA value (class c1). For both classes representing instances with marginally elevated PSA value (classes c2 and c3) the abundance of the corresponding peptides is increased with respect to the instance of class c1. It can be observed that no single features have highest discriminating power. Instead, groups of features in different regions of the spectra establish highest accuracy.

### Comparison with Existing Methods

In principle, the feature selection techniques evaluating subsets of features can be directly applied on the whole feature space. But due to the high dimensionality of the data sets, there are performance limitations for established methods. CFS as implemented in WEKA is not applicable due to memory limitations. Consistency based subset evaluation is more efficient, because it does not consider correlations among the features. It can be applied with an efficient forward selection search strategy on the full dimensional data sets. This search algorithm implemented in WEKA starts with an empty feature subset and performs greedy hillclimbing with a backtracking facility which can be controlled by a parameter. This parameter specifies the maximum number of consecutively added features if no improvement in accuracy can be achieved. As in the default settings of WEKA, this parameter is set to 5.

**Consistency-based subset evaluation.** For the ovarian data set, consistency-based subset evaluation applied on the full dimensional space finds a three dimensional attribute subset, containing the m/z values of 0.005, 2.79 and 244.66 and an accuracy of 99.60% with both, SVM and 5-NN. Although the accuracy is remarkably high, this subset of attributes provides not much information on the data set. Two of the three m/z values are of the area of very low molecular weight. This region consists of fragments of peptides that can be rather arbitrarily split up during ionization. The attribute 244.66 is the top ranked attribute by information gain. Biomarker identification for diagnosis requires identifying attribute subsets with highest predictive accuracy. But besides that, interesting regions of the spectra that show evident differences among the objects of the different classes should be identified. The result of consistency-based subset evaluation only represents two regions. On the prostate data set consistency-based subset evaluation ends up with a subset of 7 attributes and a low classification accuracy of 77.01% using SVM and 86.33% using 5-NN.

**Rankers.** For high dimensional data, ranker methods are often the only choice because of the performance limitations. However, it is critical to identify a suitable number of attributes to use for classification. For both examined methods, information gain and reliefF it turned out to be uncritical to leave out the attributes evaluated with zero. But still approximately 6,000 (for information gain on ovarian) to 14,000 (for reliefF on prostate) remain, which is still too much for interpretation, and an accuracy far below the optimum. Information gain and relief do not consider correlations and interactions among the features, thus most of the information at a certain cut-off point is redundant, whereas other regions of the spectrum providing additional information are not represented as all.

Using the method according to the present invention, groups of features were found for both data sets providing a very high sensitivity and specificity for cancer identification. This result can be used as an input for further data mining steps. The approach according to the present invention can also be adapted specifically to more-class-problems. For example, well discriminating feature sets among samples belonging to the class of early stage cancer and samples corresponding to different benign conditions may be determined.

### References

*[1] "*WEKA machine learning package, http://www.cs.waikato.ac.nz/ml/weka". Universitity ofWaikato.
[2] G. Alexe, S. Alexe, L. A. Liotta, E. Petricoin, M. Reiss, and P. L. Hammer. Ovarian cancer detection by logical analysis of proteomic data. Proteomics, 4(3):766-783, 2004.
[3] M. Betke and N. Makris. Fast object recognition in noisy images using simulated annealing. Technical Report AIM-1510, 1994.
[4] T. Conrads, V. Fusaro, S. Ross, D. Johann, V. Rajapakse, B. Hitt, S. Steinberg, E. Kohn, D. Fishman, G. Whitely, J. Barrett, L. Liotta, E. r. Petricoin, and T. Veenstra. "Highresolution serum proteomic features for ovarian cancer detection". Endocrine-Related Cancer, 11(2):163-178, 2004.
[5] U. M. Fayyad and K. B. Irani. Multi-interval discretization of continuous-valued attributes for classification learning. In IJCAI, pages 1022-1029, 1993.
[6] M. A. Hall. Correlation-based feature selection for discrete and numeric class machine learning. In ICML, pages 359-366, 2000.
[7] M. A. Hall and G. Holmes. Benchmarking attribute selection techniques for discrete class data mining. IEEE Transactions on Knowledge and Data Engineering, 15(6):1437-1447, 2003.
[8] K. Kira and L. A. Rendell. A practical approach to feature selection. In ML, pages 249-256, 1992.
[9] I. Kononenko. Estimating attributes: Analysis and extensions of relief. In ECML, pages 171-182, 1994.
[10] L. A. Liotta, M. Ferrari, and E. Petricoin. Clinical proteomics: written in blood. Nature, 425(6961):905, Oct 2003.
[11] H. Liu and R. Setiono. A probabilistic approach to feature selection - a filter solution. In ICML, pages 319-327, 1996
[12] O. C. Martin and S. W. Otto. Combining simulated annealing with local search heuristics. Technical Report CSE-94-016, 1993.
[13] T. M. Mitchell. Machine Learning. Mc Graw-Hill, 1997.
[14] M. D. Natale and J. A. Stankovic. Applicability of simulated annealing methods to real-time scheduling and jitter control. In IEEE Real-Time Systems Symposium, pages 190-199, 1995.
[15] E. Petricoin, A. Ardekani, B. Hitt, P. Levine, V. Fusaro, S. Steinberg, G. Mills, C. Simone, D. Fishman, E. Kohn, and L. Liotta. "Use of proteomic patterns in serum to identify ovarian cancer". Lancet, 359(9306):572-577, 2002.
[16] E. R. Petricoin, D. Ornstein, C. Paweletz, A. Ardekani, P. Hackett, B. Hitt, A. Velassco, T. C, W. L, W. K, C. Simone, P. Levine, W. Linehan, M. Emmert-Buck, S. Steinberg, E. Kohn, and L. LA. "Serum proteomic patterns for detection of prostate cancer.". J Natl Cancer Inst., 94(20):1576-1578, 2002.
[17] J. R. Quinlan. C4.5: Programs for Machine Learning. Morgan Kaufmann, 1993.
[18] J. S. Yu, S. Ongarello, R. Fiedler, X. W. Chen, G. Toffolo, C. Cobelli, and Z. Trajanoski. Ovarian cancer identification based on dimensionality reduction for high-throughput mass spectrometry data. Bioinformatics, 21(10):2200-2209, 2005.

## Claims

1. Method for feature selection on proteomic data derived from mass spectrometry for identifying biomarker candidates, the method comprising the steps of:
(a) removing irrelevant features using a ranker selected from the group of information gain, relief and reliefF, resulting in a first set of features and determining the classification accuracy of the first set of features using a classifier;
(b) selecting the best ranked features using a searched strategy which is a binary search, wherein the search space is divided into intervals of monotonically increasing size, so that the number of features is further reduced to result in a second set of features, wherein the classification accuracy of the second set of features is equal to or greater than the classification accuracy of the first set of features;
(c) selecting the best representative of features for a region by removing redundant features in the region among the second set of features and optionally adding features from the first set of features representing a region which is not or underrepresented by the features of the second set of features, whereby the region size is determined by using a binning function.

2. The method according to any one of the preceding claims, wherein the proteomic data is high- throughput mass spectrometry data.

3. A method for identifying cancer, in particular ovarian or prostate cancer, using the method according to any one of the preceding claims.

## Patentansprüche

1. Verfahren zur Merkmalselektion an aus einer Massenspektrometrie abgeleiteten proteomischen Daten zum Identifizieren von Biomarkerkandidaten, wobei das Verfahren die Schritte aufweist:
(a) Entfernen von irrelevanten Merkmalen mittels eines Einstufers, der aus der Gruppe Informationsgewinn, Relief und ReliefF ausgewählt ist, was einen ersten Satz von Merkmalen ergibt, und Feststellen der Klassifikationsgenauigkeit des ersten Satzes von Merkmalen mittels eines Klassifikators;
(b) Auswählen der am besten eingestuften Merkmale mittels einer Suchstrategie, die eine Binärsuche ist, wobei der Suchraum in Intervalle mit monoton zunehmender Größe unterteilt ist, so dass die Anzahl der Merkmale weiter reduziert wird, um einen zweiten Satz von Merkmalen zu ergeben, wobei die Klassifikationsgenauigkeit des zweiten Satzes von Merkmalen gleich oder größer als die Klassifikationsgenauigkeit des ersten Satzes von Merkmalen ist;
(c) Auswählen der besten repräsentativen Merkmale für einen Bereich durch Entfernen von redundanten Merkmalen in dem Bereich aus dem zweiten Satz von Merkmalen und optionales Hinzufügen von Merkmalen aus dem ersten Satz von Merkmalen, die einen Bereich repräsentieren, der nicht durch die Merkmale des zweiten Satzes von Merkmalen repräsentiert oder unterrepräsentiert wird, wodurch die Bereichsgröße mittels einer Binning-Funktion bestimmt wird.

2. Verfahren nach einem der vorhergehenden Ansprüche, wobei die proteomischen Daten Massenspektrometrie-Daten mit hohem Durchsatz sind.

3. Verfahren zum Erkennen von Krebs, insbesondere Ovarialkrebs oder Prostatakrebs mittels des Verfahrens nach einem der vorhergehenden Ansprüche.

## Revendications

1. Procédé de sélection de données protéomiques dérivées d'une spectrométrie de masse pour l'identification de biomarqueurs candidats, ledit procédé comprenant les étapes suivantes :
(a) suppression de caractéristiques non pertinentes au moyen d'un classeur sélectionné dans le groupe comprenant gain d'information, Relief et ReliefF, menant à un premier ensemble de caractéristiques, et détermination de la précision de classification du premier ensemble de caractéristiques au moyen d'un classifieur ;
(b) sélection des caractéristiques les mieux classées au moyen d'une stratégie explorée qui est une recherche binaire, l'espace de recherche étant divisé en intervalles de grandeur croissant de manière monotone, de manière à réduire encore le nombre de caractéristiques pour mener à un deuxième ensemble de caractéristiques, la précision de classification du deuxième ensemble de caractéristiques étant supérieure ou égale à la précision de classification du premier ensemble de caractéristiques ;
(c) sélection de la plus représentative des caractéristiques pour une région par suppression des caractéristiques redondantes dans la région dans le deuxième ensemble de caractéristiques et par ajout facultatif de caractéristiques du premier ensemble de caractéristiques, représentatives d'une région non représentée, ou sous-représentée par les caractéristiques du deuxième ensemble de caractéristiques, la grandeur de région étant déterminée au moyen d'une fonction de tri.

2. Procédé selon l'une des revendications précédentes, où les données protéomiques sont des données spectrométrie de masse à haut débit.

3. Procédé de détection d'un cancer, en particulier d'un cancer de l'ovaire ou d'un cancer de la prostate au moyen du procédé selon l'une des revendications précédentes.
